# Europäisches Patentamt
# European Patent Office
# Office européen des brevets

(19)

(11) Veröffentlichungsnummer : **0 075 819**
**B1**

(12) # EUROPÄISCHE PATENTSCHRIFT

(45) Veröffentlichungstag der Patentschrift :
**30.12.86**

(51) Int. Cl.⁴ : **C 07 D251/60, B 01 J 21/20**

(21) Anmeldenummer : **82108647.7**

(22) Anmeldetag : **18.09.82**

(54) Verfahren zur Verbesserung der Melaminqualität durch Reaktivierung von bei der Melaminsynthese eingesetzten Katalysatoren.

(30) Priorität : **26.09.81 DE 3138419**

(43) Veröffentlichungstag der Anmeldung :
**06.04.83 Patentblatt 83/14**

(45) Bekanntmachung des Hinweises auf die Patenterteilung : **30.12.86 Patentblatt 86/52**

(84) Benannte Vertragsstaaten :
**BE DE FR GB IT NL**

(56) Entgegenhaltungen :
**EP-A- 0 051 156**
**AT-B- 246 146**
**DE-B- 1 167 348**
**DE-B- 1 208 306**
**DE-B- 1 209 570**
**DE-B- 2 220 905**

(73) Patentinhaber : **BASF Aktiengesellschaft**
**Carl-Bosch-Strasse 38**
**D-6700 Ludwigshafen (DE)**

(72) Erfinder : **Fromm, Dieter, Dr.**
**Auf der Halde 7**
**D-6718 Gruenstadt (DE)**
Erfinder : **Schier, Ernst-Juergen, Dr.**
**Graefenthalstrasse 14**
**D-6719 Altleiningen (DE)**
Erfinder : **Schneehage, Hans Henning, Dr.**
**Plauser Strasse 10**
**D-6719 Weisenheim (DE)**
Erfinder : **Vodrazka, Wolfgang, Dr.**
**Uhlandstrasse 8**
**D-6713 Freinsheim (DE)**

**Beschreibung**

Die vorliegende Erfindung betrifft ein Verfahren zur Verbesserung der Melaminqualität durch Reaktivierung von Katalysatoren, die bei der chemisch-katalytischen Umwandlung von Harnstoff zu Melamin eingesetzt werden.

Es ist bekannt, daß bei der Melaminsynthese aus Harnstoff, die in Gegenwart von Katalysatoren bei Temperaturen von 300 bis 450 °C und bei Drucken zwischen 1 bar und 10 bar unter Zusatz von Ammoniak oder Ammoniak enthaltenden Gasen, z. B. Ammoniak und Kohlendioxid enthaltenden Gasen, wie z. B. den von Melamin befreiten, im wesentlichen aus 2 Volumenteilen Ammoniak und 1 Volumenteil Kohlendioxid bestehenden Reaktionsgasen als Trägergas durchgeführt wird, auch weniger flüchtige Nebenprodukte wie Melem, Melamincyanurat oder Cyamelursäure entstehen.

Die Katalysatorporen und -oberflächen werden in zunehmendem Maße mit diesen nichtflüchtigen Produkten belegt, so daß es im Dauerbetrieb zu einem zunehmenden Aktivitätsabfall des Katalysators kommt und das den Reaktor verlassende Gasgemisch immer größere Anteile an Isocyansäure enthält, die bei der Abkühlung der Reaktionsgase zur Abscheidung des Melamins mit diesem zu Melamincyanurat u. dgl. reagieren. Als Verunreinigungen im Melamin machen sich solche Stoffe störend bei der Herstellung von Lacken aus Melamin-Formaldehyd-Harzen bemerkbar. Da sie in unlöslicher Form und feinster Verteilung vorliegen (wie z. B. Melamincyanurat), lassen sie sich aus den Melamin-Formaldehyd-Lösungen nicht oder nur mit großem Aufwand abfiltrieren.

Durch die Einstellung eines im alkalischen Bereich liegenden pH-Wertes, z. B. durch Zusatz von Natronlauge, lassen sich zwar die in reinem Formaldehyd unlöslichen organischen Verunreinigungen des Melamins in Lösung bringen, bei manchen Rezepturen zur Herstellung von Melamin-Formaldehyd-Harzen kann jedoch nicht im alkalischen Bereich gearbeitet werden.

Zur Reaktivierung eines in seiner Aktivität nachlassenden Katalysators kann man diesen in bekannter Weise bei Reaktionstemperaturen längere Zeit mit Ammoniak spülen (DE-C-12 09 570). Dadurch kann zwar Melem in Melamin übergeführt werden, die übrigen, die Aktivität des Katalysators ebenfalls vermindernden Stoffe aber lassen sich nicht vollständig entfernen, abgesehen davon, daß diese Methode mit einem wirtschaftlich untragbaren Ammoniakverbrauch verbunden ist.

Gemäß der DE-C-12 09 570 kann dieser Nachteil dadurch vermieden werden, daß man die Reaktion in zwei Stufen ausführt und die Katalysatoren zwischen erster und zweiter Stufe von Zeit zu Zeit austauscht. Diese Verfahrensweise ist jedoch relativ aufwendig.

Gemäß der japanischen Patentanmeldung 22 385/79 werden die Katalysatoren dadurch von Verunreinigungen befreit, daß sie bei Temperaturen zwischen 380 und 800 °C mit Wasserdampf enthaltendem Sauerstoff oder Sauerstoff enthaltendem Gas in Kontakt gebracht werden und die anhaftenden organischen Substanzen weggebrannt werden. Dieses Verfahren hat den großen Nachteil, daß die Sauerstoff-Behandlung des gegebenenfalls pyrophoren Katalysators sehr kontrolliert geschehen muß, um ein Durchgehen der Reaktion, d. h. einen unkontrollierten Temperaturanstieg zu vermeiden, weil bei höheren Temperaturen die Gefahr einer dauerhaften Schädigung des Katalysators durch Gefügeänderung besteht. Ein weiterer Nachteil besteht darin, daß das Abbrennen der Verunreinigungen nicht im Reaktor selbst ausgeführt werden kann, da die für die Melaminsynthese eingesetzten Reaktoren für die dabei möglicherweise auftretenden hohen Temperaturen nicht ausgelegt sind. Um dieses Verfahren auszuführen, muß der Katalysator daher aus dem Reaktor entfernt und in einem speziellen Ofen behandelt werden. Dies bedeutet für eine großtechnische Anlage einen enormen Aufwand, so daß dieses Verfahren in der Praxis ebenfalls nicht anwendbar ist.

In der DE-B-1 167 348 und der entsprechenden AT-B-246 146 wird ein Verfahren zur Regenerierung von ausgebrauchten Katalysatoren für die Melaminherstellung beschrieben, bei dem die inaktiv gewordenen Katalysatoren mit überhitztem Wasserdampf bei Temperaturen von 250 bis 600 °C, gegebenenfalls in Gegenwart von Sauerstoff behandelt werden. Diese Behandlung kann in einer getrennten Vorrichtung oder aber auch in der Vorrichtung zur Herstellung des Melamins durchgeführt werden, wobei man anstelle des gas- oder dampfförmigen Melaminrohstoffes den überhitzten Wasserdampf durch die Katalysatorschicht leitet ; die Melaminsynthese wird somit in jedem Fall unterbrochen.

In der EP-A-0 051 156 ist ein Verfahren zum hydrolytischen Abbau von in den Reaktionsabgasen der Melaminsynthese enthaltenen Spurenbestandteilen beschrieben. In dieser Offenlegungsschrift ist weiter vermerkt, daß die Reaktionsabgase 0,1 bis 2,0 Vol-% Wasserdampf enthalten können. Es ist dieser Offenlegungsschrift weiterhin zu entnehmen, daß man die Melaminsynthese in Gegenwart von Katalysatoren und zugesetztem Ammoniak oder dieses enthaltenden Gasgemischen, z. B. den Reaktionsabgasen der Melaminsynthese selbst durchführt, wobei selbstverständlich diese Gase trocken sein müssen, da sonst eine unerwünschte, die Ausbeute an Melamin beeinträchtigende hydrolytische Spaltung des Harnstoffes eintreten würde.

In der DE-A-22 20 905 schließlich ist ein Verfahren zur katalytischen Herstellung von Melamin beschrieben, bei der der Harnstoff oberhalb der Wärmeaustauscher in die Wirbelschicht eingeführt wird. Als Katalysatoren können Oxide mit großer innerer Oberfläche wie Aluminiumoxid oder Kieselgel verwendet werden. Über die Regenerierung dieser Katalysatoren ist hier nichts erwähnt.

Der vorliegenden Erfindung lag daher die Aufgabe zugrunde, ein Verfahren zur Verbesserung der Melaminqualität durch Reaktivierung der Katalysatoren, die bei der Synthese von Melamin durch thermische Umwandlung von Harnstoff in einer Katalysatorwirbelschicht eingesetzt worden sind, durch Behandlung mit Wasserdampf enthaltenden Gasen in Abwesenheit von Sauerstoff bei erhöhten Temperaturen anzugeben, das im großtechnischen Betrieb ohne besonderen Aufwand und sicher ausgeführt werden kann.

Es wurde gefunden, daß diese Aufgabe dadurch gelöst werden kann, daß man die Behandlung während der Synthese ohne Unterbrechung der Harnstoffzufuhr bei Temperaturen von 250 bis 450 °C durchführt.

Überraschenderweise kann die erfindungsgemäße Behandlung der Katalysatoren während der Synthese des Melamins durchgeführt werden, d. h. man kann den Wasserdampf bzw. das Wasser in die Reaktionszone ohne Unterbrechung der Harnstoffzufuhr einführen. Obwohl bei dieser Arbeitsweise Harnstoff bzw. dessen Umwandlungsprodukte hydrolysiert werden, erhalten die Katalysatoren wieder ihre ursprüngliche Aktivität.

Durch die erfindungsgemäße Behandlung wird eine vollkommene Reaktivierung der Katalysatoren bewirkt, d. h. daß nach Abstellung der Wasser- bzw. Wasserdampfzufuhr entsteht sofort wieder Melamin in der ursprünglichen Ausbeute und Reinheit.

Für das erfindungsgemäße Verfahren kann man in die Wirbelschicht sowohl Wasserdampf als auch flüssiges Wasser einspeisen, da letzteres bei den bei dem erfindungsgemäßen Verfahren einzuhaltenden Temperaturen von 250 bis 450 °C, vorzugsweise von 350 bis 450 °C, ohnehin sofort verdampft. Der Wasserdampf wird mit dem für die Verwirbelung des Katalysators dienenden Gas verdünnt. In der Praxis hat es sich als zweckmäßig erwiesen, in die Wirbelschicht soviel Wasser bzw. Wasserdampf einzuspeisen, daß die Gase einen Wasserdampfgehalt von 0,5 bis 15 Vol.- % aufweisen, wobei naturgemäß bei höheren Wasserdampfgehalten die Reaktivierung schneller verläuft als bei niedrigeren Gehalten. In gleicher Weise bewirken höhere Temperaturen innerhalb des genannten Bereiches eine Beschleunigung der Reaktivierung.

Die bei der Melaminsynthese eingesetzten Katalysatoren sind üblicherweise oxidische Verbindungen, wie Kieselsäuregel, Silikate des Aluminiums, Oxide des Titans, Zirkons oder Thoriums, ferner Kaolin, Bentonit, Bauxit, Diatomeenerde, Fullererde, insbesondere aber Aluminiumoxide.

Die Dauer der erfindungsgemäßen Behandlung beträgt je nach Belegung des Katalysators, der angewandten Temperatur und der Wasserdampfkonzentration 1 bis 24 Stunden. Die erforderliche Menge Wasser bringt man vorzugsweise über die Einblasedüsen für den Harnstoff in die Wirbelschicht, während man Wasserdampf zweckmäßigerweise dem Wirbelgas zumischt.

Als Wirbelgas während der erfindungsgemäßen Behandlung dienen wie bei der Synthese selbst insbesondere aus Ammoniak- und Kohlendioxid bestehende Gase, insbesondere die Syntheseabgase, d. h. die von Melamin und Harnstoff befreiten Gase, die Ammoniak und Kohlendioxid etwa im Volumenverhältnis von 2 : 1 aufweisen.

Die erfindungsgemäße Arbeitsweise hat gegenüber den bisher bekannten Verfahren zur Regenerierung der Katalysatoren der Melaminsynthese den Vorteil, daß die Regenerierung im Synthesereaktor, d. h. ohne Aus- und Einbau des Katalysators, erfolgen kann und daß lediglich bei Nachlassen der Katalysatoraktivität, die, wie eben erwähnt, anhand der Qualität des Produktes bestimmt werden kann, während der Synthese, d. h. ohne Unterbrechung der Harnstoffzufuhr, Wasser bzw. Wasserdampf in den Synthesereaktor eingeführt wird.

Die Vorteile des erfindungsgemäßen Verfahrens seien durch die folgenden Beispiele näher erläutert:

Beispiel 1

A) In einem Wirbelschichtreaktor, der mit einem $\gamma$-Al$_2$O$_3$-Katalysator beschickt ist, werden stündlich durch Eindüsen von 6 t flüssigen Harnstoffs bei einer Reaktionstemperatur von 390 °C 2,1 t Melamin erzeugt. Der Katalysator wird mit einem Gas, bestehend aus 70 Vol.- % Ammoniak und 30 Vol.- % Kohlendioxid im Wirbelzustand gehalten. Das nach Abscheidung von Nebenprodukten und Katalysatorabrieb erhaltene dampfförmige Melamin wird durch Zufuhr von gekühltem Reaktionsgas desublimiert, durch nachgeschaltete Zyklone abgeschieden und anschließend ausgetragen.

Dieses Melamin wird auf seine Qualität untersucht, indem man 63 g Melamin in einer Mischung aus 100 ml 30 gew.- % igem Formaldehyd und 9 g destilliertem Wasser durch Erhitzen auf 90 bis 95 °C unter Rühren löst. Die Lösung wird auf Klarheit, Opaleszenz oder Trübung hin beurteilt.

B) Löst sich das wie in Beispiel 1 hergestellte Melamin ohne Zusatz von NaOH-Lösung nicht klar in Formaldehyd auf, wird dem Wirbelgas, bestehend aus ca. 70 Vol.- % NH$_3$ und ca. 30 Vol.- % CO$_2$, zusätzlich gasförmiges Ammoniak beigemischt. Die Menge des zugesetzten Ammoniaks beträgt 7,3 Vol.-% des Wirbelgases, die Zeitdauer 48 Stunden. Während dieser Zeit wird das Eindüsen von flüssigem Harnstoff in den Reaktor unterbrochen. Die Temperatur im Wirbelschichtreaktor wird auf 395 °C eingestellt.

Nach 48 Stunden wird die Reaktionstemperatur des Wirbelschichtreaktors wieder auf 390 °C eingestellt und die Produktion von Melamin aus 6 t/h flüssigem Harnstoff normal weitergeführt.

Das nun entstehende Melamin wird erneut auf seine Qualität untersucht. Es löst sich nach wie vor nicht klar in Formaldehyd auf.

C) Bildet das unter in Beispiel (1A) hergestellte Melamin mit Formaldehyd opaleszierende oder trübe Harzlösungen, die bei einem pH-Wert der Harzlösung von 8 bis 9 klar werden, kann die Produktqualität ohne Unterbrechung der Zufuhr von 6 t/h flüssigen Harnstoff zum Reaktor dadurch verbessert werden, daß man in das Wirbelgas, bestehend aus 70 Vol.-% $NH_3$ und 30 Vol.-% $CO_2$ Wasserdampf einbläst, so daß der Wasserdampfgehalt des Wirbelgases 0,8 Vol.-% beträgt.

Bei einer Einspüldauer des Wasserdampfes von 1 Stunde bei Reaktionstemperaturen von 395 °C bleibt die verbesserte Melaminqualität etwa 10 Tage lang erhalten.

**Patentansprüche**

1. Verfahren zur Verbesserung der Melaminqualität durch Reaktivierung der Katalysatoren, die bei der Synthese von Melamin durch thermische Umwandlung von Harnstoff in einer Katalysatorwirbelschicht eingesetzt worden sind durch Behandlung mit Wasserdampf enthaltenden Gasen in Abwesenheit von Sauerstoff und bei erhöhten Temperaturen, dadurch gekennzeichnet, daß man die Behandlung während der Synthese ohne Unterbrechung der Harnstoffzufuhr bei Temperaturen von 250 bis 450 °C durchführt.

2. Verfahren nach Anspruch 1, dadurch gekennzeichnet, daß man die Behandlung bei Temperaturen von 350 bis 450 °C durchführt.

**Claims**

1. A process for improving the quality of melamine by reactivating the catalysts, which have been employed in the sythesis of melamine by thermal conversion of urea in a fluidized catalyst bed, through treatment with gases containing steam, in the absence of oxygen and at elevated temperatures, wherein the treatment is carried out at from 250° to 450 °C during the synthesis, without interrupting the supply of urea.

2. A process as claimed in claim 1, wherein the treatment is carried out at from 350° to 450 °C.

**Revendications**

1. Procédé pour l'amélioration de la qualité de la mélamine par réactivation des catalyseurs qui ont été utilisés lors de la synthèse de la mélamine par transformation thermique de l'urée dans une couche de catalyseur en mouvement turbulent, par traitement avec des gaz contenant de la vapeur d'eau, en l'absence d'oxygène et à températures élevées, caractérisé par le fait que l'on effectue le traitement pendant la synthèse, sans interruption de l'introduction d'urée, à des températures de 250 à 450 °C.

2. Procédé selon la revendication 1, caractérisé par le fait que l'on effectue le traitement à des températures de 350 à 450 °C.